# EUROPEAN PATENT APPLICATION

(11) **EP 1 964 915 A1**
(43) Date of publication of application: **03.09.2008**
(21) Application number: 06835134.5
(22) Date of filing: 18.12.2006
(51) Int. Cl.: C12M 1/00, C12N 5/06, C12Q 1/02, G01N 33/15, G01N 33/50

(54) **BLOOD-BRAIN BARRIER IN VITRO MODEL, PATHOLOGIC BLOOD-BRAIN BARRIER IN VITRO MODEL, DRUG SCREENING METHOD USING THE SAME, PATHOLOGIC BLOOD-BRAIN BARRIER FUNCTION ANALYSIS METHOD AND PATHOGENESIS ANALYSIS METHOD**

(30) Priority: 19.12.2005 JP 2005364636
(71) Applicant: Pharmaco-Cell Company LTD, Nagasaki-shi Nagasaki 8528523 (JP); Niwa, Masami, Nagayo-cho, Nishi-sonogi-gun Nagasaki 8512127 (JP)
(72) Inventor: NIWA, Masami, Nishi-sonogi-gun, Nagasaki8512127 (JP); NAKAGAWA, Shinsuke, Nagasaki-shi, Nagasaki 8528032 (JP); DELI, Maria, Anna, H-H6725 (HU)
(74) Representative: Luderschmidt, Schüler & Partner
(86) International application number: PCT/JP2006/325671
(87) International publication number: WO 2007/072953

(57) **Abstract**

It is intended to provide a screening system for a centrally acting drug transported across the blood-brain barrier, a drug acting on the blood-brain barrier itself, or a drug transferred into the brain without being expected to centrally act. Moreover, another object of the present invention is to achieve pathogenesis analysis study or the screening in a diseased state by applying various diseased environments to this screening system. The present invention provides an in-vitro model of blood-brain barrier obtained by using a three-dimensional culture apparatus comprising: a culture solution; a plate holding the culture solution; and a filter immersed in the culture solution and placed in no contact with the inside bottom of the plate, the filter having plural pores of 0.35 to 0.45 µm in diameter, and by comprising: seeding primary cultured brain capillary endothelial cells onto the upper surface of the filter; seeding primary cultured brain pericytes onto the under surface of the filter; seeding primary cultured astrocytes onto the inside surface of the plate; and coculturing these cells in a normal culture solution.

## Description

### Technical Field

The present invention relates to an in-vitro model of blood-brain barrier (generally called "BBB" as an abbreviation) and an in-vitro model of diseased blood-brain barrier.

"In vitro" is a Latin term meaning "in glass" and refers to, as a biological term, a state in which a portion of a living organism is extracted and found free "outside the living organism" for various study purposes (see Iwanami Biological Dictionary, Iwanami Shoten, Publishers, Nov. 10, 1998).

Specifically, the present invention relates to a screening system for a centrally acting drug transported across the blood-brain barrier, a drug acting on the blood-brain barrier itself, or a drug transferred into the brain without being expected to centrally act.

Moreover, the present invention relates to a drug screening method using the in-vitro model of blood-brain barrier and the in-vitro model of diseased blood-brain barrier. Specifically, the present invention achieves pathogenesis study analysis or the screening in a diseased state by applying various diseased environments to the in-vitro model of blood-brain barrier or the screening system of the present invention.

### Background Art

The blood-brain barrier (BBB) is an environmental maintenance mechanism that has evolved for the survival and activity of nerve cells in a unique environment of the brain where the delicate nerve cells live. BBB can be referred to as a high functional differentiation system in which three cells, brain capillary endothelial cells, astrocytes, and pericytes, as its constitutional units functionally overcome, in cooperation, the antinomy between the entry blockage (barrier) and uptake (transport) of substances from blood to the central nervous system. The barrier function of BBB is typified by a tight junction as the cell-cell adhesion structure of brain capillary endothelial cells. The selective uptake (transport) mechanism is typified by glucose transporter type 1 (GLUT1) for D-glucose as an energy source of nerve cells. The barrier function and the transport mechanism are closely related to each other. For example, P-glycoproteins, structural members of a group of transport molecules (ABC transporters), are responsible as an efflux pump for the barrier function, while the tight junction is also responsible for the transport of low-molecular-weight compounds selective for cations rather than anions (paracellular pathway). The functional essence of BBB is the presence of the closely arranged tight junction as well as the asymmetric distribution (polarity) of carriers (transporters) responsible for selective uptake or transport molecules responsible for transcellular pathway (e.g., ion channel) in cell membranes on a lumen side (apical membrane lipid) and on a basement membrane side (basolateral membrane lipid).

This unique BBB function maintains nerve cell homeostasis. However, its presence becomes a major obstacle to some artificial treatment of living organisms for causes such as diseases. This presence is a major obstacle to the development of preventive or therapeutic drugs for central nervous diseases (dementia, Alzheimer's disease, prion disease, stroke, etc.) which is urgently needed. Most of drug candidate compounds whose effects have been found in vitro or in cell culture experiments cannot be transferred into the brain due to BBB, when administered to living organisms such as experimental animals. Thus, such compounds are not available as actual therapeutic drugs, and their development must be abandoned.

On the other hand, multi-drug therapies are currently in the mainstream. In this case, it is also expected that drugs considered to be hardly transferred into the brain are transferred into the brain through drug interaction and causes central side effects.

Thus, the prediction of drug permeability of BBB is very important. Nevertheless, the law of constant transfer into the brain is absent due to the unique function of BBB, and drug permeability cannot be determined in advance from the chemical structure or molecular weight of the drug. Study on drug transfer into the brain in animal experiments requires enormous labors and expenses. In addition, it is difficult to correctly determine only the complicated in-vivo drug transfer. Moreover, the amount of drugs used in animal experiments needs to be lager than that in in-vitro experiments. The securing of the amount of lead compounds that can bear animal experiments requires additional times and expenses and thus, is not realistic. Accordingly, the development of novel drugs acting in the brain and appropriate drug therapies require predicting drug permeability using an in-vivo reproduction system having function analogous to that of our complicated in-vivo BBB. An examination kit, if any, for conveniently testing transfer into the brain promotes the efficiency of drug discovery research which is currently conducted with enormous expenses and times and can achieve a more quick-response and reliable screening system for drug discovery.

Furthermore, the in-vitro BBB model has a big advantage. A diseased state can be reproduced easily by optimizing its culture condition. Brain capillary endothelial cells, which surround the central nerve and protect homeostasis, are associated with various central nervous diseases. Diseases for which BBB dysfunction is closely involved in their onset or diseased state progression have been elucidated recently one after another. For example, the blood-brain barrier (BBB) is a site responsible for brain edema onset. The disruption of the tight junction permits plasma proteins to be leaked out into the brain, resulting in water or electrolyte accumulation attributed to the dysfunction of transporters for Na+, glutamic acid, or the like or the dysfunction of aquaporin water channels or the like to cause the progression of the diseased state (vasogenic edema). In the analysis of BBB function in a diseased state, an in-vitro BBB model that reproduces complicated in-vivo BBB works effectively, as in the screening of drug transfer into the brain described above. Specifically, a diseased environment can be reproduced easily by changing the culture environment of the in-vitro BBB model. Such an in-vitro model of diseased BBB can be applied to pathogenesis analysis study.

Moreover, the drug permeability of BBB in a diseased state is considered to be varied from normal one. Thus, it is also important to determine drug permeability in a diseased state.

The anatomical essence of the blood-brain barrier is tightly united brain capillary endothelial cells. It was shown that astrocytes or pericytes are closely involved in the function and maintenance thereof. It has been reported that the in-vitro coculture of brain capillary endothelial cells with astrocytes enhances function specific to the blood-brain barrier function, such as a rise in the activity of alkaline phosphatase or γ-glutamyl transpeptidase (enzyme specific for the brain capillary endothelial cells), a rise in membrane resistance, the enhancement of tight junction, and the expression of P-glycoproteins (Sobue et al., 1999; Maxwell et al., 1987; Stanness et al., 1997; and Fenart et al., 1998). This indicates the importance of astrocytes. On the other hand, the roles of pericytes in the blood-brain barrier are mostly unknown. However, it has been reported in recent years that pericytes play an important role in BBB.

It has been reported that the in-vitro coculture of capillary endothelial cells with pericytes enhances membrane resistance (Dente et al., 2001). This report indicates the importance of pericytes as elements constituting the maintenance mechanism of the blood-brain barrier. Specifically, endothelial cells alone cannot form the high functional differentiation system BBB. Only the crosstalk among three cells, endothelial cells, astrocytes, and pericytes, can probably constitute the BBB. Thus, a model requires considering these three cells. However, previously reported in-vitro BBB models are only incomplete models such as a monolayer culture system of endothelial cells or a coculture system of endothelial cells with astrocytes.

Recent study results have revealed a tight junction constituent protein group as functional molecules of BBB. This group is localized on the lumen side of capillary endothelial cells (apical membrane lipid) and is the essence of the barrier function. Among them, claudin (Furuse et al.), occludin (Furuse et al.), and ZO (zonula occludens)-1 have been demonstrated to be major proteins of the barrier function. The BBB tight junction is a barrier that separates different spaces, that is, peripheral tissues (within the brain capillary) and the central nervous system (within the brain parenchyma). The tight junction is positioned at the boundary between cell membranes on the lumen side of brain capillary endothelial cells (apical membrane lipid) and on a basement membrane side (basolateral membrane lipid) facing different environments and contributes to the asymmetric arrangement of functional molecules such as transporters (polarity, the fence function of the tight junction). Moreover, the tight junction is a functional molecule unit responsible for the paracellular transport of low-molecular-weight compounds selective for cationic substances, while being a barrier. In fact, MUPP1 (multi-PDZ domain protein), a novel PDZ protein which mediates the binding with intracellular signaling molecules, has been discovered. The tight junction is not a static fixed barrier as previously believed and is being shown to be a dynamic/mobile functional molecule such as an intracellular signaling mechanism. Specifically, the maintained tight junction function is indispensable for an in-vitro BBB model. However, it is questionable whether immortalized brain capillary endothelial cells, which have been used previously because of their convenience, maintain this tight junction function. Moreover, it is considered that the immortalized brain capillary endothelial cells have, in addition to such questionable barrier function, no cell polarity and insufficient cell response to drugs. In fact, a study using rat immortalized brain capillary endothelial cells demonstrated that the tight junction proteins do not accumulate between the cells and have a very low electrical resistance value. Accordingly, a blood-brain barrier model obtained using immortalized brain capillary endothelial cells is an incomplete model and does not reflect normal BBB.

On the other hand, another reported model is obtained by adding cAMP known as a factor accelerating the tight junction function of endothelial cells to a cell culture solution for maintaining this function and is a less-than-physiological model. Particularly, such a model presents a problem in the screening of drugs that vary cAMP.

Moreover, brain capillary endothelial cells, which surround the central nerve and protect homeostasis, are associated with various central nervous diseases. Diseases for which BBB dysfunction is closely involved in their onset or diseased state progression have been elucidated recently one after another. It is also important here that the in-vivo blood-brain barrier is constituted by brain capillary endothelial cells, pericytes, and astrocytes, and each of these cells has various influences in various diseased states. It is considered that incomplete BBB models having not all of BBB constituent cells, such as a culture system of endothelial cells alone or a coculture system of endothelial cells with astrocytes, do not sufficiently reproduce BBB in a diseased state.

### Disclosure of the Invention

Accordingly, an object of the present invention is to provide a screening system for a centrally acting drug transported across the blood-brain barrier, a drug acting on the blood-brain barrier itself, or a drug transferred into the brain without being expected to centrally act. Moreover, another object of the present invention is to achieve pathogenesis analysis study or the screening in a diseased state by applying various diseased environments to this screening system.

The present inventor has conducted diligent study and has successfully developed a blood-brain barrier model that is analogous to the in-vivo blood-brain barrier and has a high tight junction by coculturing primary cultured brain capillary endothelial cells, pericytes, and astrocytes.

The objects are attained by an in-vitro model of blood-brain barrier of the present invention according to claim 1, that is, an in-vitro model of blood-brain barrier obtained by using a three-dimensional culture apparatus comprising: a culture solution; a plate holding the culture solution; and a filter immersed in the culture solution and placed in no contact with the inside bottom of the plate, the filter having plural pores of 0.35 to 0.45 µm in diameter, and by comprising: seeding primary cultured brain capillary endothelial cells onto the upper surface of the filter; seeding primary cultured brain pericytes onto the under surface of the filter; seeding primary cultured astrocytes onto the inside surface of the plate; and coculturing these cells in a normal culture solution.

Moreover, the objects are also attained by an in-vitro model of diseased blood-brain barrier of the present invention according to claim 2, that is, an in-vitro model of diseased blood-brain barrier obtained by using a three-dimensional culture apparatus comprising: a culture solution; a plate holding the culture solution; and a filter immersed in the culture solution and placed in no contact with the inside bottom of the plate, the filter having plural pores of 0.35 to 0.45 µm in diameter, and by comprising: seeding primary cultured brain capillary endothelial cells onto the upper surface of the filter; seeding primary cultured brain pericytes onto the under surface of the filter; seeding primary cultured astrocytes onto the inside surface of the plate; and coculturing these cells in a culture solution corresponding to a predetermined diseased condition.

A preferred embodiment of the present invention discloses, as described in claim 3, an evaluation method for the permeability of a drug across the blood-brain barrier, the method using an in-vitro model of blood-brain barrier according to claim 1 and comprising: adding a drug to a portion above a filter; and measuring the amount of the drug leaked out to a portion below the filter after a certain period of time.

An alternative preferred embodiment of the present invention discloses, as described in claim 4, an evaluation method for drug screening, the method using an in-vitro model of blood-brain barrier according to claim 1 and comprising: adding a drug to a portion above a filter; collecting brain capillary endothelial cells after a certain period of time; evaluating the properties of the brain capillary endothelial cells after the addition of the drug; and comparing these properties with those of the brain capillary endothelial cells before addition.

A further alternative preferred embodiment of the present invention discloses, as described in claim 5, an analysis method for the diseased state of diseased blood-brain barrier, the method comprising respectively comparing cultured brain capillary endothelial cells, brain pericytes, and astrocytes in an in-vitro model of diseased blood-brain barrier according to claim 2 with cultured brain capillary endothelial cells, brain pericytes, and astrocytes in an in-vitro model of blood-brain barrier according to claim 1.

A further alternative preferred embodiment of the present invention discloses, as described in claim 6, an evaluation method for drug reactivity in diseased blood-brain barrier, the method comprising respectively comparing cultured brain capillary endothelial cells, brain pericytes, and astrocytes in an in-vitro model of diseased blood-brain barrier according to claim 2 with cultured brain capillary endothelial cells, brain pericytes, and astrocytes in an in-vitro model of blood-brain barrier according to claim 1.

A further alternative preferred embodiment of the present invention discloses, as described in claim 7, an evaluation method for drug transfer to the brain through diseased blood-brain barrier, the method comprising respectively comparing cultured brain capillary endothelial cells, brain pericytes, and astrocytes in an in-vitro model of diseased blood-brain barrier according to claim 2 with cultured brain capillary endothelial cells, brain pericytes, and astrocytes in an in-vitro model of blood-brain barrier according to claim 1.

### (Operation)

In the present invention according to claim 1, the crosstalk among three cells were achieved by: seeding brain capillary endothelial cells onto the upper surface of a porous filter; seeding pericytes onto the under surface of the filter; and seeding astrocytes onto the inside surface of a plate serving as a receiver for the filter. As a result, an in-vitro model of blood-brain barrier analogous to the in-vivo blood-brain barrier was completed.

Moreover, in the present invention according to claim 2, an in-vitro model of diseased blood-brain barrier was obtained by applying various diseased environments to the in-vitro model of blood-brain barrier.

The present invention provides an in-vitro BBB model most analogous to the in-vivo blood-brain barrier and as such, efficiently achieves the development of a central nervous agent (centrally acting drug transported across the blood-brain barrier) which is currently urgently needed.

Moreover, the present invention provides a screening system for a drug transferred into the brain without being expected to centrally act and as such, can contribute to the development of appropriate drug therapies or the development of novel drugs. Moreover, the present invention is useful in the screening of a drug acting on the blood-brain barrier itself.

Furthermore, the present invention applies various diseased environments to this screening system and as such, achieves pathogenesis analysis study or the screening in a diseased state.

### Brief Description of the Drawings

Figure 1 is an illustrative diagram showing various coculture preparation methods;
Figure 2 is a microscopic photograph for comparing primary cultured brain capillary endothelial cells with immortalized brain capillary endothelial cells;
Figure 3 is a graph showing the effects of cAMP on RBEC and GP8.3;
Figure 4 is a graph showing variation per day of transendothelial electrical resistance value measurement results of 7 coculture models prepared by a method shown in Example 4;
Figure 5 is a graph comparing transendothelial electrical resistance value measurement results among 7 coculture models prepared by a method shown in Example 4;
Figure 6 is a photograph showing the expression of tight junction constituent proteins (occludin, claudin-5, and ZO-1) in 7 coculture models prepared by a method shown in Example 4;
Figure 7 is a graph showing an electrical resistance value of an ischemia-reperfusion model;
Figure 8 is a graph showing the exacerbation of ischemia-reperfusion injury (reduction in electrical resistance value) attributed to coculture;
Figure 9 is a graph showing the exacerbation of ischemia-reperfusion injury (increase in Na-F permeability) attributed to coculture;
Figure 10 is a graph showing the effects of a radical scavenger edaravone on an ischemia-reperfusion BBB model; and
Figure 11 is a schematic diagram of an embodiment of an in-vitro model of blood-brain barrier of the present invention.

### Best Mode for Carrying Out the Invention

An in-vitro model of blood-brain barrier of the present invention is obtained by coculturing primary cultured brain capillary endothelial cells, primary cultured brain pericytes, and primary cultured astrocytes in a three-dimensional culture apparatus comprising a porous filter. The in-vitro model of blood-brain barrier of the present invention has a highly developed tight junction and serves as a blood-brain barrier reconstruction system more analogous to the in-vivo blood-brain barrier.

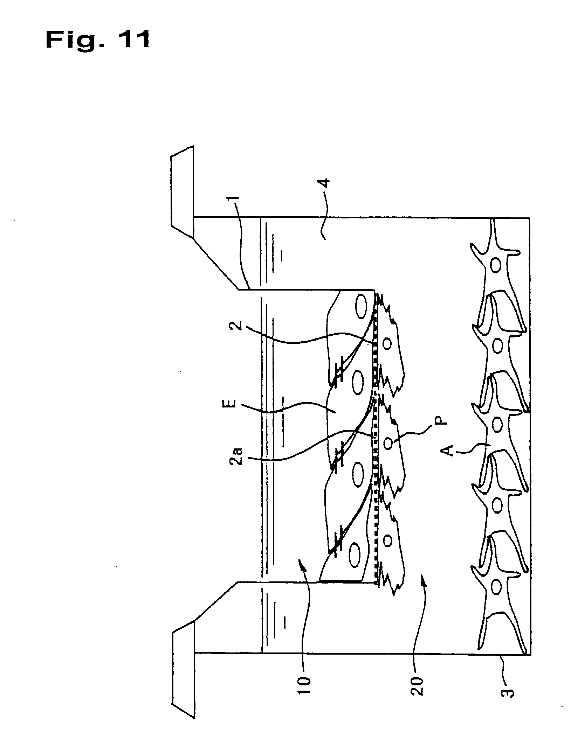

Figure 11 is a schematic diagram of an embodiment of an in-vitro model of blood-brain barrier of the present invention. A container 3 contains a culture solution 4. A filter 2 is immersed at a predetermined position in the culture solution 4 by a hanger 1 that hangs the filter 2. A portion above the filter 2 having plural pores 2a of 0.4 µm in diameter is intended to represent a vascular lumen side 10. A portion below the filter 2 is intended to represent a brain parenchyma side 20. Specifically, a structure that probably reproduces the in-vivo blood-brain barrier most closely is obtained by: seeding primary cultured brain capillary endothelial cells E onto the surface of the filter 2; seeding primary cultured brain pericytes P onto the under surface of the filter 2; and further seeding primary cultured astrocytes A onto a portion below the filter 2. Moreover, the porosity of the filter 2 achieves the crosstalk among three cells and achieves the reproduction of even the maintenance/regulation mechanism of the complicated in-vivo blood-brain barrier.

The highly developed tight junction, which is a characteristic of BBB, can be induced in the endothelial cells by three-cell coculture to provide a blood-brain barrier reconstruction system that can bear drug screening and the like. The use of this blood-brain barrier reconstruction system can easily determine whether a drug expected to act in the brain or a drug transferred into the brain without being expected to centrally act can be transported across the blood-brain barrier. Specifically, such determination can be conducted by: adding the drug to the portion above the filter 2 serving as the vascular lumen side 10; and measuring the amount of the drug leaked out to a portion below the filter 2 after a certain period of time. Likewise, the use of this blood-brain barrier reconstruction system can easily perform the screening of a drug acting on the blood-brain barrier itself. Specifically, such determination can be conducted by: adding the drug to the portion above the filter 2 serving as the vascular lumen side 10; and comparing the properties of the brain capillary endothelial cells E between before and after the addition of the drug after a certain period of time.

Furthermore, a diseased BBB kit can be prepared easily by changing the culture condition of the BBB kit. Specifically, a diseased BBB kit is prepared by changing the culture solution 4 of the BBB kit to an actual diseased condition. The analysis of the diseased state of BBB and the determination of drug reactivity in a diseased state or drug transfer to the brain can be achieved by comparing changes in brain capillary endothelial cells E, pericytes P, and astrocytes A in the diseased BBB kit from those cultured in a normal culture solution.

### Example 1

### (Isolation of brain capillary slice)

The brain of a three-week-old rat was excised within a clean bench and placed in an ice-cold Phosphate buffer saline (-/-) (PBS (-/-); Sigma). The dura mater, the cerebellum, the interbrain, the brain stem, and the like were removed on a sterilized filter paper to leave only the cerebral cortex. This cerebral cortex was placed in 3 mL of an ice-cold Dulbecco's Modified Eagle's Medium (DMEM; manufactured by Sigma) and finely cut into a size of approximately 1 mm³ with a surgical knife. 15 mL of DMEM containing enzymes (collagenase-class 2 (1 mg/ml; manufactured by Worthington) and 300 µL of DNase (15 µg/mL; manufactured by Sigma)) was further added thereto. The mixed solution was moved up and down 25 times with a 5-mL pipette to suspend a pellet. After shaking/incubation at 37°C for 90 minutes, 10 mL of DMEM was added thereto, and the mixture was centrifuged. The precipitated pellet after centrifugation was centrifuged with 20% BSA (manufactured by Sigma)/DMEM. The pellet in the lower layer was suspended in 15 mL of DMEM containing enzymes (collagenase/dispase (1 mg/mL; manufactured by Boehringer Mannheim)) and DNase (6.7 µg/mL). The suspension was shaken/incubated at 37°C for 60 minutes. Then, 10 mL of DMEM was added thereto, and the mixture was centrifuged. The pellet in the lower layer was layered on Percoll (33%; manufactured by Pharmacia) centrifuged in advance at 3000 g for 1 hour, and the mixture was centrifuged. The endothelial layer was collected with a syringe and washed twice with DMEM to obtain a brain capillary slice. The resulting separated brain capillary slice was seeded onto a dish coated with collagen (0.1 mg/ml; manufactured by Sigma) and fibronectin (0.1 mg/mL; manufactured by Sigma). The brain capillary endothelial cells were cultured at 37°C for 2 days in 5% CO₂/95 atmosphere in a culture solution (RBEC culture solution 1) containing 20% Plasma-derived serum (PDS)-DMEM/F12 supplemented with bFGF (1 mg/mL; manufactured by Boehringer Mannheim), heparin (100 µg/mL; manufactured by Sigma), gentamicin (50 µg/mL; manufactured by Sigma), and puromycin (4 µg/mL; manufactured by Sigma). On the 3rd day, the culture solution was replaced by a puromycin-free culture solution (culture solution containing 20% PDS-DMEM/F12 supplemented with bFGF (1 mg/mL), heparin (100 µg/mL), gentamicin (50 µg/mL); RBEC culture solution 2) to continue culture until 80% confluence.

### Example 2

### (Brain capillary pericytes)

The brain capillary slice of Example 1 contains a few percents of pericytes. Thus, the brain capillary slice was seeded onto a dish coated with collagen and cultured at 37°C for 1 week in 5% CO₂/95% atmosphere in a culture solution containing 10% fetal bovine serum (FBS)-DMEM supplemented with gentamicin (50 µg/mL) to promote pericyte growth. In this stage, the brain capillary endothelial cells are mixed with the pericytes. Next, the cells were detached with a 1 x trypsin-EDTA solution (manufactured by Sigma) and seeded again onto a non-coated dish to isolate the pericytes (the endothelial cells cannot adhere to the dish, while only the pericytes grow therein).

### Example 3

### (Astrocytes)

The brain was removed from a 1- or 2-day-old rat within a clean bench and placed in ice-cold PBS (-/-). The dura mater, the cerebellum, the interbrain, the brain stem, and the like were removed on a sterilized filter paper to leave only the cerebral cortex. This cerebral cortex was placed in a 50-mL tube, and 10 mL of ice-cold DMEM was added thereto. The mixed solution was slowly moved up and down with a 20-G syringe to break the tissue apart. The solution was left standing for a while. Then, 5 mL of the supernatant was placed in another 50-mL tube. 5 mL of DMEM was further added to 5 mL of the remaining cell suspension, and the mixed solution was moved up and down with a 20-G syringe. This procedure was repeated until the cell mass could not be observed visually. The last 5 mL of the cell suspension was also added thereto. Then, the cell suspension was passed through Cell Strainer (registered trademark; manufactured by Falcon) of 70µm. The cells were collected by centrifugation and resuspended in 10% FBS-DMEM. The cells were seeded onto a 75-cm² flask. The cells were cultured at 37°C in 5% CO₂/95% atmosphere in a culture solution containing 10% FBS-DMEM supplemented with gentamicin (50 µg/mL). After cell confluence, the flask was shaken at 200 rpm for 1 hour. The resulting floating microglia was removed. The cells were continuously cultured in a 1 x trypsin-EDTA solution and seeded again onto a new 75-cm² flask. After cell confluence, the flask was shaken again at 200 rpm for 1 hour. The resulting floating microglia was removed to obtain astrocytes.

### Example 4

### (Coculture preparation method)

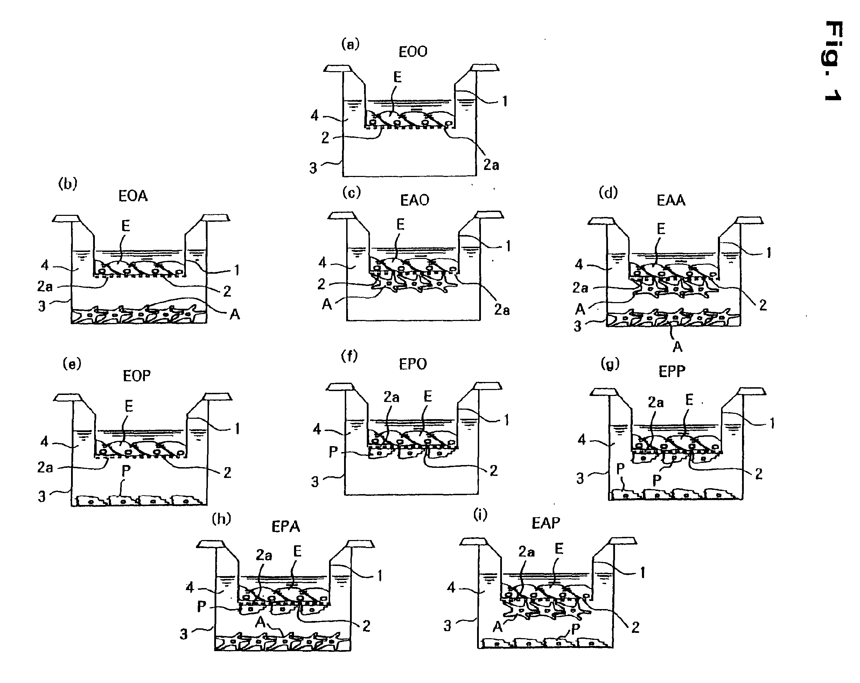

Figure 1 is an illustrative diagram showing various coculture preparation methods. The brain capillary endothelial cells obtained in Example 1, the pericytes, and the astrocytes were prepared into the following various coculture models using Transwell (registered trademark; manufactured by Corning, 0.4 µm in pore size):
(i) Monolayer culture system of brain capillary endothelial cells E (E00)
   Both surfaces of a polyester membrane of the Transwell (registered trademark) insert were coated with collagen (0.1 mg/ml) and fibronectin (0.1 mg/mL). The Transwell (registered trademark) insert was mounted in a well of 12-well culture plate (manufactured by Corning). Then, the brain capillary endothelial cells E (1.5 x 10⁵ cells/cm²) were seeded onto the inside of the Transwell (registered trademark) insert and cultured at 37°C in 5% CO₂/95% atmosphere in the RBEC culture solution 2.
(ii) Coculture system of brain capillary endothelial cells E in no contact with astrocytes A (EOA)
   The inside bottom of a 12-well culture plate was coated with a poly-L-lysine (manufactured by Sigma) solution. The astrocytes A (1.0 x 10⁵ cells/cm²) were seeded thereonto and cultured at 37°C for a whole day and night in 5% CO₂/95 atmosphere in a culture solution containing 10% FBS-DMEM supplemented with gentamicin (50 µg/mL). On the next day, the culture solution was replaced by the RBEC culture solution 2. The brain capillary endothelial cells E were seeded onto the inside of the Transwell (registered trademark) insert by the method shown in the paragraph (i).
(iii) Coculture system of brain capillary endothelial cells E in contact with astrocytes A (EAO)
   Both surfaces of a polyester membrane of the Transwell (registered trademark) insert were coated with collagen (0.1 mg/ml) and fibronectin (0.1 mg/mL). The polyester membrane was mounted in a dish with the membrane turned upside down. The astrocytes A (2.0 x 10⁴ cells/cm²) were seeded thereonto and cultured for 6 hours or longer. The Transwell (registered trademark) insert was mounted in a well of a 12-well culture plate. The cells were cultured at 37°C for a whole day and night in 5% CO₂/95% atmosphere in a culture solution containing 10% FBS-DMEM supplemented with gentamicin (50 µg/mL). On the next day, the culture solution was replaced by the RBEC culture solution 2. The brain capillary endothelial cells E were seeded onto the inside of the Transwell (registered trademark) insert by the method shown in the paragraph (i).
(iv) Coculture system of brain capillary endothelial cells E in no contact with pericytes P (EOP)
   The inside bottom of a 12-well culture plate was coated with a collagen solution. The pericytes P (1.0 x 10⁵ cells/cm²) were seeded thereonto and cultured at 37°C for a whole day and night in 5% CO₂/95 atmosphere in a culture solution containing 10% FBS-DMEM supplemented with gentamicin (50 µg/mL). On the next day, the culture solution was replaced by the RBEC culture solution 2. The brain capillary endothelial cells E were seeded onto the inside of the Transwell (registered trademark) insert by the method shown in the paragraph (i).
(v) Coculture system of brain capillary endothelial cells E in contact with pericytes P (EPO)
   Both surfaces of a polyester membrane of the Transwell (registered trademark) insert were coated with collagen (0.1 mg/ml) and fibronectin (0.1 mg/mL). The polyester membrane was mounted in a dish with the membrane turned upside down. The pericytes (2.0 x 10⁴ cells/cm²) were seeded thereonto and cultured for 6 hours or longer. The Transwell (registered trademark) insert was mounted in a well of a 12-well culture plate. The cells were cultured at 37°C for a whole day and night in 5% CO₂/95 atmosphere in a culture solution containing 10% FBS-DMEM supplemented with gentamicin (50 µg/mL). On the next day, the culture solution was replaced by the RBEC culture solution 2. The brain capillary endothelial cells E were seeded onto the inside of the Transwell (registered trademark) insert by the method shown in the paragraph (i).
(vi) Coculture system of brain capillary endothelial cells E in contact with pericytes P and in no contact with astrocytes A (EPA)
   The inside bottom of a 12-well culture plate was coated with a poly-L-lysine solution. The astrocytes A (1.0 x 10⁵ cells/cm²) were seeded thereonto and cultured at 37°C for a whole day and night in 5% CO₂/95 atmosphere in a culture solution containing 10% FBS-DMEM supplemented with gentamicin (50 µg/mL). At the same time, both surfaces of a polyester membrane of the Transwell (registered trademark) insert (0.4 µm in pore size) were coated with collagen (0.1 mg/ml) and fibronectin (0.1 mg/mL). The polyester membrane was mounted in a dish with the membrane turned upside down. The pericytes P (2.0 x 10⁴ cells/cm²) were seeded thereonto and cultured for 6 hours or longer. The Transwell (registered trademark) insert was mounted in a well of the 12-well culture plate containing the astrocytes A seeded thereon. The cells were cultured at 37°C for a whole day and night in 5% CO₂/95 atmosphere in a culture solution containing 10% FBS-DMEM supplemented with gentamicin (50 µg/mL). On the next day, the culture solution was replaced by the RBEC culture solution 2. The brain capillary endothelial cells E were seeded onto the inside of the Transwell (registered trademark) insert by the method shown in the paragraph (i) .
(vii) Coculture system of brain capillary endothelial cells E in contact with astrocytes A and in no contact with pericytes P (EAP)
   The inside bottom of a 12-well culture plate was coated with a collagen solution. The pericytes P (1.0 x 10⁵ cells/cm²) were seeded thereonto and cultured at 37°C for a whole day and night in 5% CO₂/95% atmosphere in a culture solution containing 10% FBS-DMEM supplemented with gentamicin (50 µg/mL). At the same time, both surfaces of a polyester membrane of the Transwell (registered trademark) insert (0.4 µm in pore size) were coated with collagen (0.1 mg/ml) and fibronectin (0.1 mg/mL). The polyester membrane was mounted in a dish with the membrane turned upside down. The astrocytes A (2.0 x 10⁴ cells/cm²) were seeded thereonto and cultured for 6 hours or longer.
   The Transwell (registered trademark) insert was mounted in a well of the 12-well culture plate containing the astrocytes A seeded thereon. The cells were cultured at 37°C for a whole day and night in 5% CO₂/95 atmosphere in a culture solution containing 10% FBS-DMEM supplemented with gentamicin (50 µg/mL). On the next day, the culture solution was replaced by the RBEC culture solution 2. The brain capillary endothelial cells E were seeded onto the inside of the Transwell (registered trademark) insert by the method shown in the paragraph (i) .

### Example 5

### (Comparison of immortalized brain capillary endothelial cells with primary cultured brain capillary endothelial cells)

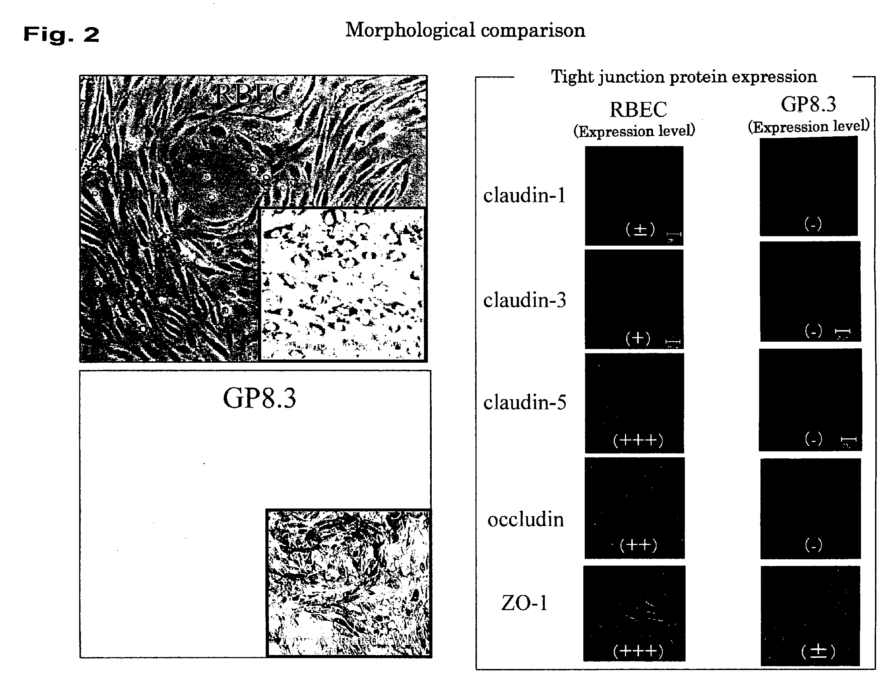

### 5-1. Expression of von Willebrand factor (vWF) in primary cultured brain capillary endothelial cells and immortalized brain capillary endothelial cells (Figure 2)

RBEC obtained in Example 1 for cell culture and GP8.3 obtained in Example 4 were seeded at each cell density of 1 x 10⁵ cells/cm² onto an 8-well culture slide. After 3 days, the culture solution was removed, and the slide was washed twice with PBS (-/-). After washing, the cells were fixed in 3% paraformaldehyde at room temperature for 10 minutes. Then, the cells were treated with a 0.2% triton-X solution at room temperature for 10 minutes. The cells were treated with a 3% H₂O₂ solution for 3 minutes to remove endogenous peroxidase activity. The slide was washed twice with PBS (-/-) and then blocked with a 3% BSA-PBS (-/-) solution at room temperature for 30 minutes. The slide was washed with 0.1% BSA-PBS (-/-) and then incubated at 37°C for 30 minutes using anti-vWF (Sigma) for primary antibody reaction. The slide was washed three times with 0.1% BSA-PBS (-/-) and then incubated at 37°C for 30 minutes using biotinylated secondary antibodies for secondary antibody reaction. Then, the slide was washed three times with 0.1% BSA-PBS (-/-) and then reacted with a peroxidase-labeled streptavidin solution at room temperature for 10 minutes. Finally, diaminobenzidine tetrahydrochloride was used as a chromogenic substrate. The cells were observed with a microscope. Figure 2 is a microscopic photograph for comparing the primary cultured brain capillary endothelial cells with the immortalized brain capillary endothelial cells. Both RBEC and GP8.3 exhibited positivity for vWF serving as a marker for endothelial cells.

### 5-2. Expression of tight junction constituent proteins in primary cultured brain capillary endothelial cells and immortalized brain capillary endothelial cells (Figure 2)

RBEC obtained in Example 1 for cell culture and GP8.3 obtained in Example 4 were seeded at each cell density of 1 x 10⁵ cells/cm² onto an 8-well culture slide and cultured for 3 days. The culture solution was removed, and the slide was washed twice with PBS (-/-). After washing, the cells were fixed in 3% paraformaldehyde at room temperature for 10 minutes. Then, the cells were treated with a 0.2% triton-X solution at room temperature for 10 minutes. The slide was washed twice with PBS (-/-) and then blocked with a 3% BSA-PBS (-/-) solution at room temperature for 30 minutes. The slide was washed with 0.1% BSA-PBS (-/-) and then incubated at 37°C for 30 minutes using antibodies (anti-claudin-1; Zymed, anti-claudin-3; Zymed, anti-claudin-5; Zymed, anti-occludin; BD Bioscience, and anti-ZO-1; Zymed) specific for each protein for primary antibody reaction. The slide was washed three times with 0.1% BSA-PBS (-/-) and then incubated at 37°C for 30 minutes using Alexa 488-conjugated IgG antibodies for secondary antibody reaction. Then, the slide was washed three times with 0.1% BSA-PBS (-/-). Then, the cells were observed with LSM 5 Pascal (Zeiss). Figure 2 is a microscopic photograph for comparing the primary cultured brain capillary endothelial cells with the immortalized brain capillary endothelial cells. As a result, RBEC was confirmed to have the accumulation of all the tight junction constituent proteins between the cells, whereas GP8.3 could be confirmed to have the slight accumulation of only ZO-1.

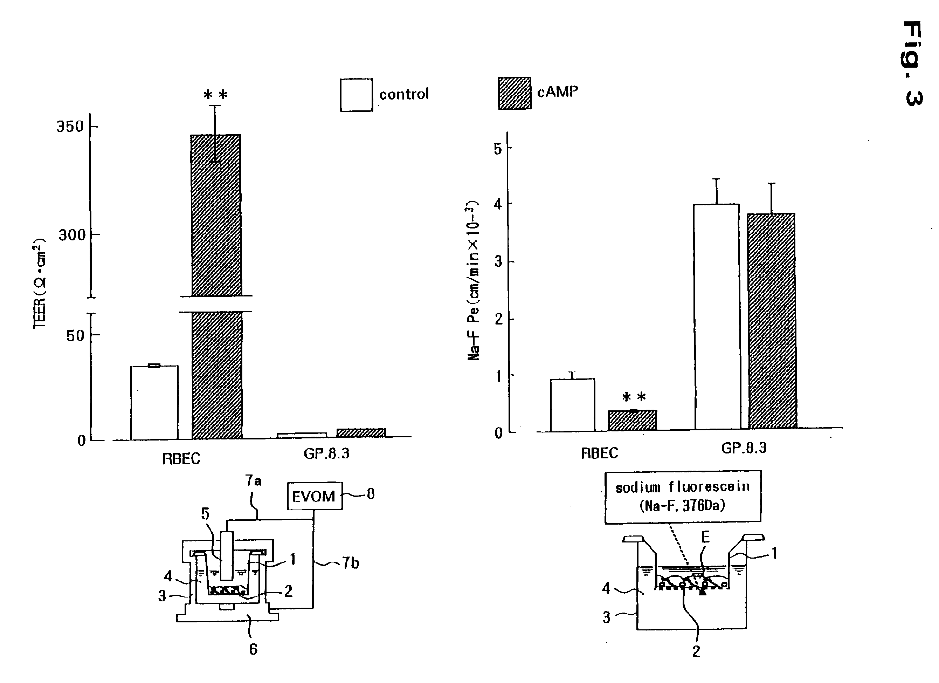

### 5-3. Study on electrical resistance (TEER) and Na-F permeability of primary cultured brain capillary endothelial cells and immortalized brain capillary endothelial cells (Figure 3)

To study whether RBEC and GP8.3 cells retain tight junction function, an experiment described below was conducted. Moreover, cAMP, which is known as a substance accelerating tight junction function, was applied thereto, and the reaction was also studied. RBEC obtained in Example 1 for cell culture and GP8.3 obtained in Example 4 were seeded at each cell density of 1 x 10⁵ cells/cm² onto 12-well type Transwell (registered trademark) and cultured for 3 days. On the 2nd day, cAMP (cpt-cAMP; manufactured by Sigma) was applied thereto at a concentration of 250 µM, and the cells were treated for 12 hours. TEER was measured with ENDOHM (manufactured by WPI). Then, the experiment on Na-F permeability was conducted. First, the culture solution was removed, and the plate was washed twice with PBS (-/-). Then, a portion (brain parenchyma side) below the insert in the well was filled with 1.5 mL of an assay buffer: PBS (+/+); Sigma, d-glucose (4.5 mg/mL); manufactured by Wako Pure Chemical Industries, Ltd., and HEPES (10 mM); Sigma. An assay buffer (0.5 mL) containing 10 µg/mL sodium-fluorescein (Na-F); Sigma was added to a portion (vascular side) above the insert. Then, the insert was transferred to a new well every 20 minutes. The fluorescence intensity of the sample was measured (excitation wavelength: 485 nm, fluorescence wavelength: 530 nm) using a fluorophotometer (Shimadzu Corp.). An Na-F concentration was calculated from a calibration curve. Clearance and permeability coefficients (P) were calculated according to the methods of Dehouck and Isobe et al. (Dehouck et al., 1992; and Isobe et al., 1996). The clearance was calculated according to the equation: Clearance (µL)=[C]A x VA/[C]L wherein the amount of Na-F transferred from the chamber on the vascular side to the chamber on the brain parenchyma side is indicated in µL; [C]L represents the initial concentration of Na-F placed on the vascular side; [C]A represents the final concentration of Na-F and EBA transferred to the brain parenchyma side; and VA represents the volume (1.5 mL) of the chamber on the brain parenchyma side. The permeability coefficients (cm/min.) were determined according to the equation:
1/PSapper=1/PSmembrane+1/PStrans. In the equation, PS represents (permeability coefficient x (the surface area of the membrane) by the slope of a line of clearance plotted against time. Papp represents an apparent permeability coefficient. Ptrans represents a true permeability coefficient. Pmembrane represents the permeability coefficient of only the membrane. Figure 3 is a graph showing the effects of cAMP on RBEC and GP8.3. As a result, RBEC was demonstrated to have electrical resistance significantly higher than that of GP8.3 and Na-F permeability lower than that of GP8.3. Moreover, the tight junction function-accelerating effects of cAMP observed in RBEC were not confirmed in GP8.3.

### Example 6

### Study on coculture using primary cultured brain capillary endothelial cells, primary cultured brain pericytes, and astrocytes

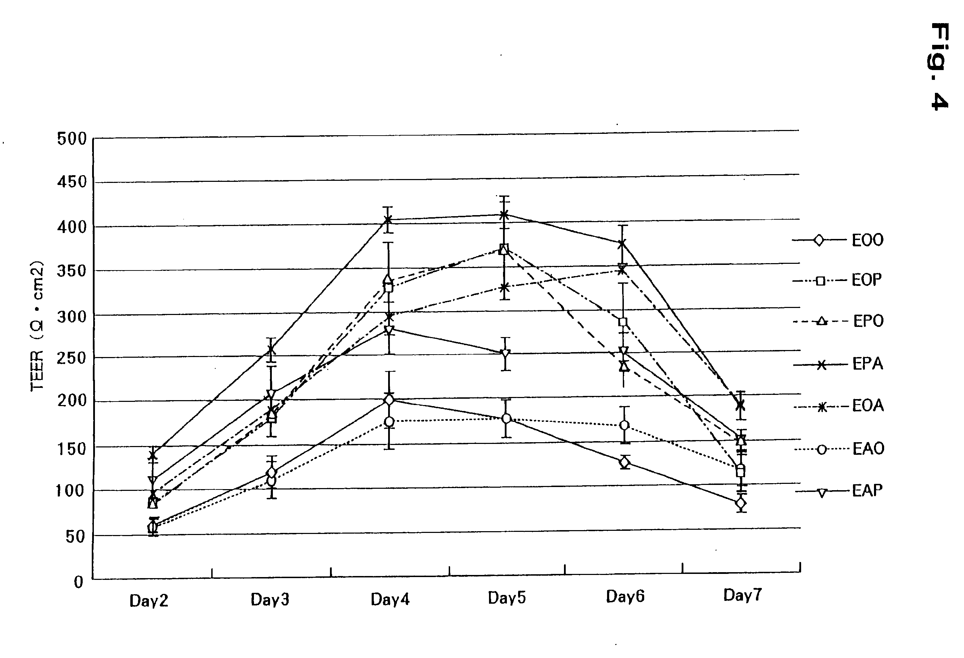

### 6-1. Transendothelial electrical resistance (Figure 4)

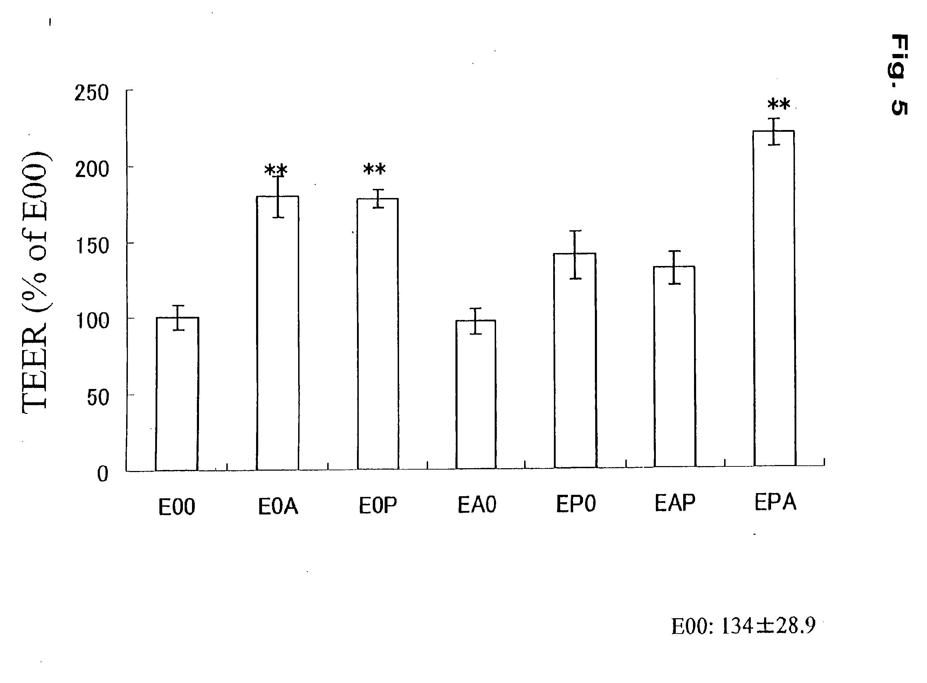

Figure 4 is a graph showing variation per day of transendothelial electrical resistance value measurement results of 7 coculture models prepared by the method shown in Example 4. Figure 5 is a graph comparing transendothelial electrical resistance value measurement results among 7 coculture models prepared by the method shown in Example 4. Transendothelial Electrical Resistance (TEER) was measured with ENDOHM (manufactured by WPI). The electrical resistance value of each BBB model was measured from 2 to 7 days after model preparation. The TEER values of E00, EOA, and EOP were calculated by subtracting therefrom the electrical resistance value of only the membrane. The TEER values of EA0 ad EAP were calculated by subtracting therefrom the electrical resistance value of the membrane on which only the astrocytes were cultured. The TEER values of EPO ad EPA were calculated by subtracting therefrom the electrical resistance value of the membrane on which only the pericytes were cultured. The coculture system of brain capillary endothelial cells in no contact with astrocytes (EOA), the coculture system of brain capillary endothelial cells in no contact with pericytes (EOP), and the coculture system of brain capillary endothelial cells in contact with pericytes and in no contact with astrocytes (EPA) were confirmed to have a significant rise in transendothelial electrical resistance as compared with the monolayer culture system of brain capillary endothelial cells (E00). Among them, the EPA type exhibited the highest value.

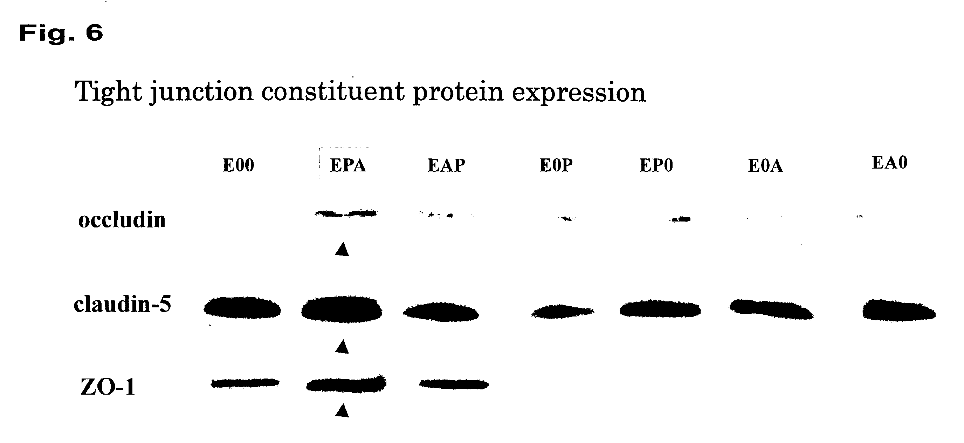

### 6-2. Western blotting (Figure 6)

Figure 6 is a photograph showing the expression of tight junction constituent proteins (occludin, claudin-5, and ZO-1) in 7 coculture models prepared by the method shown in Example 4. Each BBB model was prepared using 6-well Transwell (registered trademark). After 3 days, the culture solution was removed, and the plate was washed twice with PBS (-/-). After washing, to remove the cells adhering to the under side of the membrane, the cells on the under surface were peeled off with a surgical knife. Then, 300 µL of a cytolytic solution containing CelLytic-M (Sigma) supplemented with a protease inhibitor cocktail (Sigma) was added thereto, and the plate was incubated on a shaker for 20 minutes. Then, the cytolytic solution was collected and centrifuged at 12000 x g for 15 minutes. The supernatant was collected. A portion thereof was used for protein amounts, and the remaining portion was used as a sample. A Tris-HCl-5 x sample buffer containing SDS and 2-mercaptoethanol was added to the sample. The sample was treated at 95°C for 5 minutes and stored at -80°C. The protein amount of each sample was measured. The proteins were separated by SDS-polyacrylamide electrophoresis (SDS-PAGE) so that the protein amounts were equal among the samples. Next, the proteins were transferred to a PVDF membrane, and the membrane was blocked at room temperature for 1 hour with a 0.1% Tween 20-TBS (100 mM NaCl and 10 mM Tris-HCl) solution containing 1% perfect block (registered trademark; MoBiTec). The membrane was incubated at room temperature for 1 hour using antibodies (anti-claudin-1; Zymed, anti-occludin; BD Bioscience, anti ZO-1; Zymed, and β-actin; Sigma) specific for each protein for primary antibody reaction. The membrane was washed for 5 minutes x three times with 0.1% Tween 20-TBS and then incubated for 1 hour using horseradish peroxidase-conjugated anti-IgG antibodies for secondary antibody reaction. The membrane was washed for 5 minutes x three times with 0.1% Tween 20-TBS. Then, the bands of interests were detected with ECL plus (Amersham). The bands were analyzed using NIH imaging. Among the coculture systems, the EPA type exhibited the highest acceleration of occludin, claudin-5, and ZO-1 expression.

### Example 7

### Ischemia-reperfusion BBB model

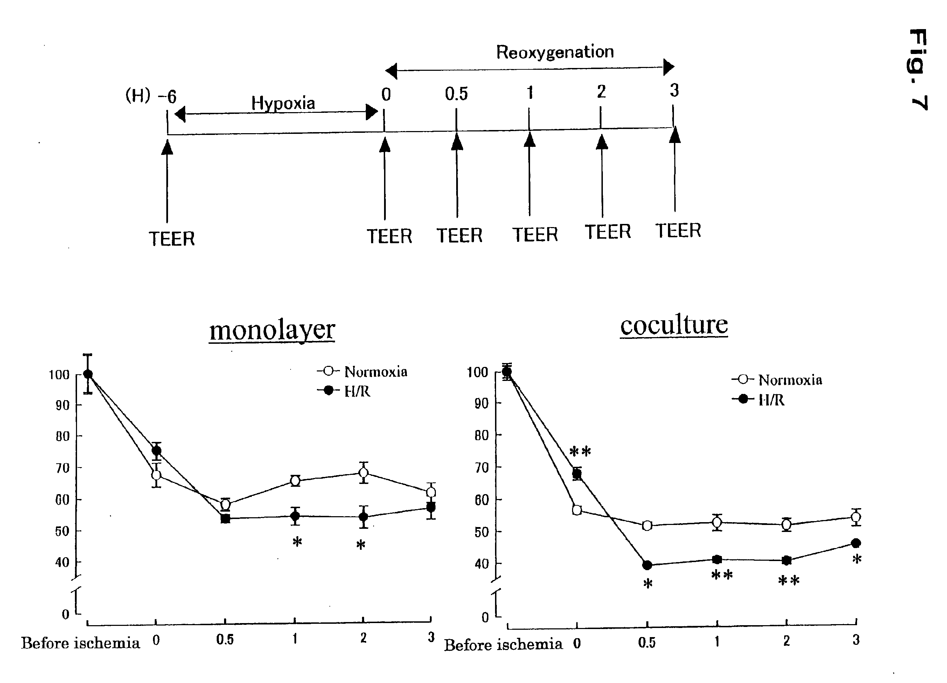

### 7-1. Transendothelial electrical resistance and Na-F permeability (Figures 7, 8, and 9)

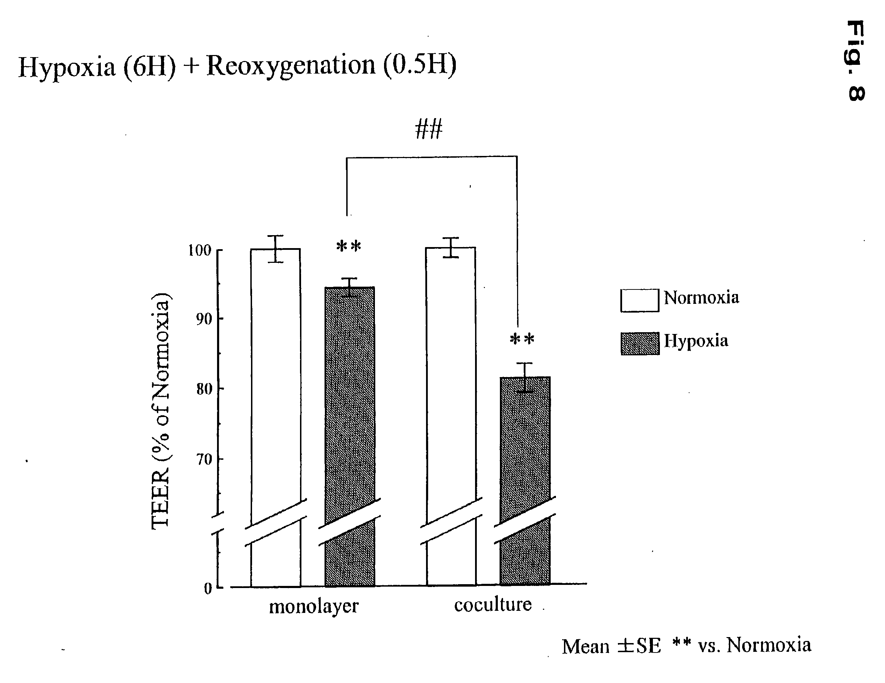

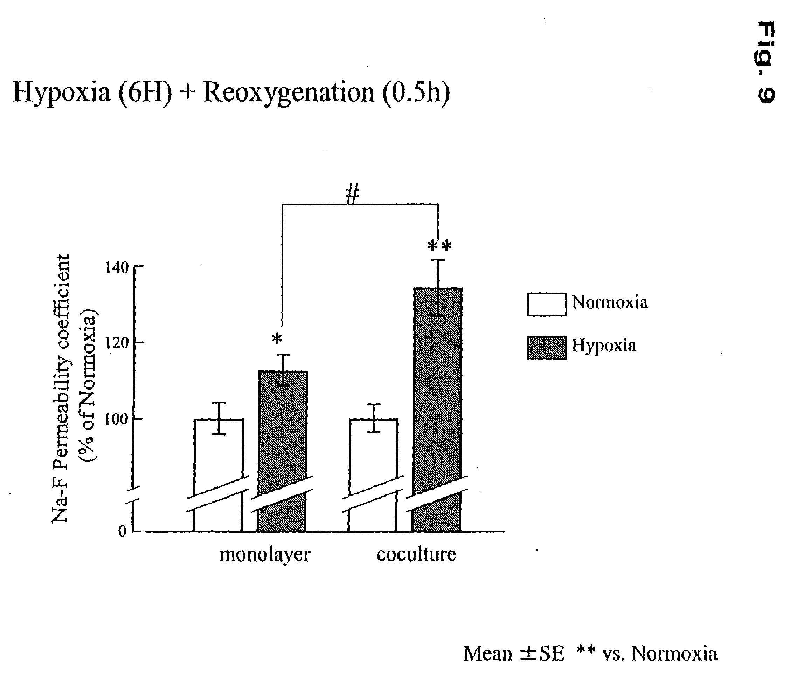

A monolayer culture system of brain capillary endothelial cells (E00) and a coculture system of brain capillary endothelial cells in contact with pericytes and in no contact with astrocytes (EPA) were prepared using the coculture methods shown in the paragraphs (i) and (vi) of Example 5 for cell culture. The prepared models were washed once with PBS (-/-). Then, the culture solution was replaced by a glucose-free Krebs-Ringer buffer (143 mM NaCl, 4.7 mM KCl, 1.3 mM CaCl₂, 1.2 mM MgCl₂, 1.0 mM NaH₂PO₄, 25 mM NaHCO₃, and 11 mM sucrose (all from Wako Pure Chemical Industries, Ltd.), and 10 mM HEPES (Sigma), pH 7.4) bubbled with nitrogen gas for 5 minutes. These models were placed in Anaerocult (registered trademark) A mini and incubated for 6 hours (ischemia period). After incubation, the solution was replaced by a normal Krebs-Ringer buffer (143 mM NaCl, 4.7 mM KCl, 1.3 mM CaCl₂, 1.2 mM MgCl₂, 1.0 mM NaH₂PO₄, 25 mM NaHCO₃, 11 mM d-glucose (Sigma), and 10 mM HEPES, pH 7.4). The models were incubated for 3 hours (reperfusion period). For a control, all the procedures were performed at 37°C in 95% air/5% CO₂ using a normal Krebs-Ringer buffer. The electrical resistance values of the ischemia-reperfusion models are shown in Figure 7. The ischemia-reperfusion group was observed to have reduction in electrical resistance and increase in Na-F permeability as compared with the control group and demonstrated to exhibit reduced tight junction function. Moreover, this reduction in tight junction function was remarkably observed in the EPA group rather than the E00 group, suggesting that pericytes and astrocytes influence ischemia-reperfusion injury (Figures 8 and 9).

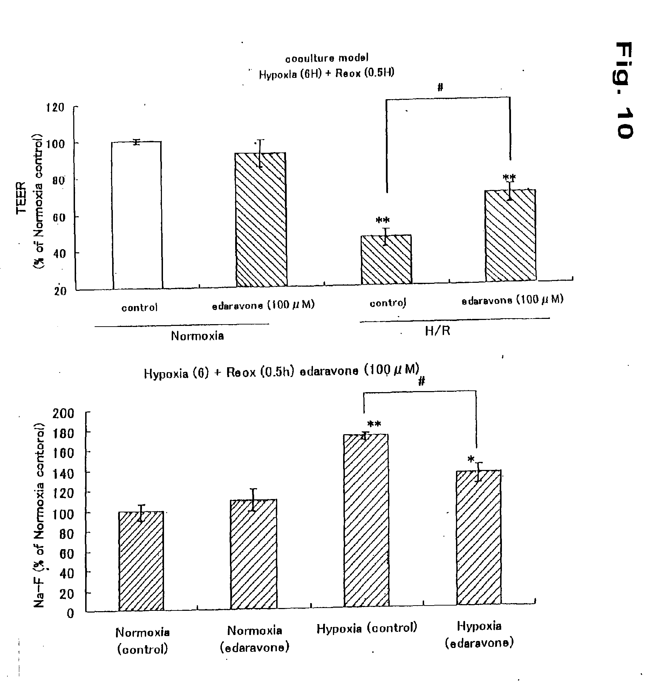

### 7-2. Effects of radical scavenger edaravone on ischemia-reperfusion BBB model (Figure 10)

Figure 7 is a graph showing the effects of a radical scavenger edaravone on an ischemia-reperfusion BBB model. Ischemia-reperfusion BBB models were prepared by the method, and the effects of edaravone was studied. The radical scavenger edaravone was added thereto at the end of the ischemia period. As shown in Figure 10, the administration of edaravone significantly suppressed reduction in tight junction function (reduction in TEER and rise in Na-F permeability) attributed to ischemia-reperfusion.

## Claims

1. An in-vitro model of blood-brain barrier obtained by using a three-dimensional culture apparatus comprising: a culture solution; a plate holding the culture solution; and a filter immersed in the culture solution and placed in no contact with the inside bottom of the plate,
the filter having plural pores of 0.35 to 0.45 µm in diameter, and by comprising: seeding primary cultured brain capillary endothelial cells onto the upper surface of the filter; seeding primary cultured brain pericytes onto the under surface of the filter; seeding primary cultured astrocytes onto the inside surface of the plate; and coculturing these cells in a normal culture solution.

2. An in-vitro model of diseased blood-brain barrier obtained by using a three-dimensional culture apparatus comprising: a culture solution; a plate holding the culture solution; and a filter immersed in the culture solution and placed in no contact with the inside bottom of the plate,
the filter having plural pores of 0.35 to 0.45 µm in diameter, and by comprising: seeding primary cultured brain capillary endothelial cells onto the upper surface of the filter; seeding primary cultured brain pericytes onto the under surface of the filter; seeding primary cultured astrocytes onto the inside surface of the plate; and coculturing these cells in a culture solution corresponding to a predetermined diseased condition.

3. An evaluation method for the permeability of a drug across the blood-brain barrier, the method using an in-vitro model of blood-brain barrier according to claim 1 and comprising: adding a drug to a portion above a filter; and measuring the amount of the drug leaked out to a portion below the filter after a certain period of time.

4. An evaluation method for drug screening, the method using an in-vitro model of blood-brain barrier according to claim 1 and comprising: adding a drug to a portion above a filter; collecting brain capillary endothelial cells after a certain period of time; evaluating the properties of the brain capillary endothelial cells after the addition of the drug; and comparing these properties with those of the brain capillary endothelial cells before addition.

5. An analysis method for the diseased state of diseased blood-brain barrier, the method comprising respectively comparing cultured brain capillary endothelial cells, brain pericytes, and astrocytes in an in-vitro model of diseased blood-brain barrier according to claim 2 with cultured brain capillary endothelial cells, brain pericytes, and astrocytes in an in-vitro model of blood-brain barrier according to claim 1.

6. An evaluation method for drug reactivity in diseased blood-brain barrier, the method comprising respectively comparing cultured brain capillary endothelial cells, brain pericytes, and astrocytes in an in-vitro model of diseased blood-brain barrier according to claim 2 with cultured brain capillary endothelial cells, brain pericytes, and astrocytes in an in-vitro model of blood-brain barrier according to claim 1.

7. An evaluation method for drug transfer to the brain through diseased blood-brain barrier, the method comprising respectively comparing cultured brain capillary endothelial cells, brain pericytes, and astrocytes in an in-vitro model of diseased blood-brain barrier according to claim 2 with cultured brain capillary endothelial cells, brain pericytes, and astrocytes in an in-vitro model of blood-brain barrier according to claim 1.
